# EUROPÄISCHE PATENTANMELDUNG

(11) **EP 2 465 566 A1**
(43) Veröffentlichungstag der Anmeldung: **20.06.2012**
(21) Anmeldenummer: 10195596.1
(22) Anmeldetag: 17.12.2010
(51) Int. Cl.: A61M 21/00, A61N 5/06, F21S 10/00

(54) **Verwendung optischer Signale zur Beeinflussung eines physiologischen und/oder psychologischen Vorgangs**

(71) Anmelder: Zaitchik, Semen, 10365 Berlin (DE)
(72) Erfinder: Zaitchik, Semen, 10365 Berlin (DE)
(74) Vertreter: Lange, Sven

(57) **Zusammenfassung**

Die Erfindung beschreibt ein System zur Beeinflussung eines physiologischen und/oder psychologischen Vorgangs, bei dem optische Signale auf einer Projektionsfläche rhythmisch und sinuswellenartig wiedergegeben werden, wobei die Änderung der optischen Signale subsensorisch mit einer definierten Frequenz erfolgt.

## Beschreibung

Die Erfindung betrifft ein System physiologischen und/oder psychologischen Vorgangs, wobei optische Signale auf eine Projektionsfläche wiedergegeben werden, und die optischen Signale rhythmisch und sinuswellenartig geändert werden.

Im Stand der Technik sind Verfahren bekannt, die allgemein als Bioresonanztherapie bezeichnet werden und zur Behandlung von Allergien, Migräne, Schlafstörungen, chronischen Schmerzen und weiteren Krankheiten verwendet werden. Bei der Bioresonanz beziehungsweise den entsprechenden Geräten, werden Elektroden mit mindestens zwei Stellen der Haut eines Probanden in Verbindung gebracht. Im einfachsten Fall nimmt der Proband zwei bewegliche Elektroden in jeweils eine Hand. Die elektrischen Signale interferieren mit körpereigenen elektrischen Signalen, wodurch ggf. krankhafte elektromagnetische Schwingungen aufgehoben werden können. Dies hat unter Umständen einen positiven Effekt auf eine Krankheit des Probanden.

Ein ähnlicher Effekt kann bei der Anwendung von Edelsteinen, Metallen und Farbkarten beobachtet werden, die auch über heilsame Schwingungen verfügen.

Im Stand der Technik ist die Chronophysiologie bekannt. Die Chronophysiologie beschäftigt sich mit der zeitlichen Organisation von Lebewesen. Sie berücksichtigt dabei psychologische Rhythmen, die sich in Verbindung mit dem Faktor Zeit verändern. Ein psychologischer Rhythmus kann beispielsweise für einen bestimmten Menschen festlegen, zu welchen Tageszeiten er die beste Aufmerksamkeit zum Lernen hat, oder welcher Schlaf-Wach-Rhythmus sich optimal auf sein Wohlbefinden auswirkt. Es ist beispielsweise möglich, ein Profil seiner täglichen Leistungsfähigkeit zu erstellen. Dazu werden physiologische Körperfunktionen, mentale Kriterien (wie Aufmerksamkeit, intellektuelle Leistungsfähigkeit) und deren zeitliche Veränderung erfasst. Chronopsychologie ist eine Erweiterung der Chronobiologie und der Chronomedizin. Dabei werden "äußere Einflüsse" wirksam, wie Schichtarbeit, Zeitumstellung durch Flugreisen (sog. Jet-Lag), Sommerzeit/Winterzeit. Ebenso werden "innere Einflüsse" wirksam, wie biologische Rhythmen, altersbedingte Veränderungen, oder ob der Mensch eher ein Morgen- oder Abendtyp (Chronotypen) ist. Psychologische Rhythmen eines Menschen werden beeinflusst durch die Situation, die Tätigkeit und durch individuelle Faktoren des einzelnen Menschen. Die Biorhythmologie ist ebenfalls eine Wissenschaft, die im Stand der Technik beschrieben ist und sich mit dem Biorhythmus befasst. Der Biorhythmus ist eine Erscheinung bei Organismen, der bestimmte Lebensvorgänge in einer festen Periodisierung ablaufen lässt. Man spricht dann auch von einer biologischen oder biochemischen Oszillation. Die Vorgänge müssen selbsterregt (endogen) sein, um von einem Biorhythmus sprechen zu können. Er bildet beim Menschen, bei den Tieren und den Pflanzen die Grundlage für die Chronobiologie. Die Periodizität bzw. Phasen sind bei einzelnen Organismen von unterschiedlicher Dauer. Jeder Organismus richtet sich nach dem Tagesrhythmus, der durch die Erddrehung bestimmt wird. Doch haben Untersuchungen gezeigt, dass die innere Uhr des Menschen nur etwa 23 Stunden für einen Tag anzeigt. Andere Perioden werden z.B. durch Ebbe und Flut (Gezeiten; je 12,8 Stunden) bestimmt, durch die Mondphase mit 28 Tagen (lunar) oder 14-15 Tagen (semi-lunar), durch die Jahreszeiten (unter anderem Winterschlaf bei Tieren, Vogelzug, Blattabwurf bei Pflanzen, Eisprung, Wanderung der Aale und Lachse) und durch klimatische Faktoren, die sehr unterschiedlich sein können. Einige Periodizitäten konnten bisher aber noch nicht gedeutet werden.

Im Stand der Technik sind Verfahren offenbart, die der Beeinflussung des Biorhythmus des Menschen dienen. So beschreibt beispielsweise die DE 1 954 929 7 C2 ein Verfahren und eine Vorrichtung zur Beeinflussung der menschlichen Psyche. Hierbei wird die Augenbewegung einer Person elektrisch im Augenbereich durch Sensoren erfasst und die Messwerte an ein Auswertungssystem übertragen. Das Auswertungssystem bewertet hiernach den psychischen Zustand der Person und steuert ein Stimulationssystem, was in Abhängigkeit der Bewertung des psychischen Zustands der Person Frequenz und/oder Amplituden modulierte akustische Grundmuster oder Farbsignale als Stimulationssignale appliziert.

Durch solche Verfahren soll die psychische Belastung, die durch erhöhte berufliche und private Beanspruchung hervorgerufen wird, kompensiert werden. Die psychische Belastung, die im alltäglichen Sprachgebrauch als Stress bezeichnet wird, beeinflusst nicht nur die Leistungsfähigkeit einer Person, sondern auch den Gesundheitszustand dieser.

Um die psychische Belastung einer Person zu kompensieren, ist weiterhin in der DE 102 004 031 459 A1 ein Verfahren beschrieben, bei der eine Person ein Bild für eine Entspannung vorgelegt wird. Das Bild ändert sich ständig, wobei die Geschwindigkeit der Veränderung des Bildes so eingestellt wird, dass der Betrachter diese Veränderung bei ständiger Betrachtung des Bildes nicht feststellt. Das Bild erscheint der Person in jedem einzelnen Augeblick als nicht verändert. Hierdurch soll eine Entspannung erreicht werden, die für Therapie- und Übungszwecke verwendet werden kann. Im Stand der Technik ist beschrieben, dass die minimale Geschwindigkeit einer fließenden Bewegung von Objekten auf einem Hintergrund aus anderen Objekten, bei der diese Bewegung von einem Betrachter noch wahrgenommen werden kann, ein bis zwei Winkelsekunden pro Sekunde beträgt.

Weiterhin beschreibt die DE 1 996 156 8 A1 ein Verfahren zum Steigern des psychischen und physischen Wohlbefindens und zur Therapie bestimmter Krankheitsbilder. Das Verfahren wird vorzugsweise in Kombination mit einer Sauerstofftherapie angewendet, bei der Sauerstoff mittels einer Atemmaske inhaliert wird. Dem Anwender werden hierbei audiovisuelle Eindrücke mittels einem Kopfhörer und einer speziellen Brille, die beide mit einer Mindmaschine verbunden sind, übermittelt. Die Sauerstofftherapie wird angewandt, um bei bekannten Krankheitsbildern eine Verbesserung zu erzielen. Außerdem kann die Konzentrations- und Leistungsfähigkeit sowie die Minderung von Stresssymptomen erzielt werden. Der einzuatmende Sauerstoff ist vorzugsweise ionisiert, d.h. mit negativen Ionen angereichert. Während der Sauerstofftherapie trägt der Anwender einen Kopfhörer und eine Brille, die beide mit einer sogenannten mind-machine verbunden sind. In diesem Gerät werden die Impulse und Signale erzeugt, die in dem Kopfhörer und der Brille zu den akustischen und optischen Reizen und Eindrücken umgewandelt werden. Der Anwender erfährt somit eine Sauerstofftherapie und gleichzeitig erlebt er die Eindrücke einer mind-machine, die zu einer mentalen Entspannung führt.

Für die Anwendung der im Stand der Technik beschriebenen Verfahren und Methoden muss ein Anwender aktiv an dem Verfahren teilnehmen und es bedarf eines hohen apparativen Aufwandes. Nachteilig ist außerdem, dass keine Stimulation über einen langen Zeitraum möglich ist, da die Belastung für die Person zu hoch ist. Außerdem ist eine impulsartige Stimulation mittels einer mind-machine nicht oder nur schwer mit einem Rhythmus eines Organismus zu vereinbaren, da es sich bei der impulsartigen Stimulation um keinen natürlich vorkommenden Rhythmus handelt. Weiterhin muss der Patient oder Proband an spezielle Geräte oder Apparaturen angeschlossen werden, was die Verwendbarkeit und die Anwendungsmöglichkeiten erheblich einschränkt.

Aufgabe der Erfindung war es demgemäß, ein Verfahren bereitzustellen, was nicht die Nachteile oder Mängel des Standes der Technik aufweist.

Gelöst wird die Aufgabe durch die unabhängigen Ansprüche, vorteilhafte Ausführungsformen finden sich in den abhängigen Ansprüchen.

Es war völlig überraschend, dass ein System zur Beeinflussung eines physiologischen und/oder psychologischen Vorgangs bereitgestellt werden kann, was nicht die Nachteile oder Mängel des Standes der Technik aufweist, wobei das System eine optische Signale wiedergebende Projektionsfläche umfasst, die so ausgerichtet ist, dass ein Patient oder ein Anwender die optischen Signale auf dieser bewusst oder unbewusst wahrnimmt und wobei sich die optischen Signale rhythmisch und sinuswellenartig ändern, wobei die Änderung insbesondere mit einer Frequenz von 6 Sekunden bis 12 Stunden, bevorzugt 10 Sekunden bis 15 Minuten, erfolgen. Die Änderungen erfolgen insbesondere subsensorisch. Es war völlig überraschend, dass die Änderungen der Signale so langsam geschehen, dass ein Mensch in einem normalen Zustand diese Änderungen nicht bemerken kann. Es handelt sich bevorzugt um eine subsensorische Wahrnehmung. Die Erfindung eröffnet somit einen neuen technischen Bereich, da im Stand der Technik lediglich Verfahren mit Biofeedback beschrieben sind, bei denen die Patienten durch optische Signale beeinflusst werden, wobei die optischen Signale bemerkbar sind. In einem Bereich von 6 Sekunden bis 12 Stunden kann sich ein optisches Signal sehr langsam entfalten, so dass die Änderung für einen Betrachter unbemerkt bleibt. In einer besonders bevorzugten Ausführungsform erfolgt die Änderung mit einer Frequenz von 6 Sekunden bis 1 Stunde, was insbesondere einen Effekt auf Atmung, hypothalamische, nebennierenrindische und physiologische Aktivität sowie Eiweißstoffsynthese und Zellmembran Durchlässigkeit aufweist.

Vorteilhafterweise kann das zur subsensorischen Stimulation eines Patienten oder eines Betrachters verwendet werden. Die bevorzugt unbewusste Wahrnehmung ermöglicht die Nutzung ohne speziell organisierte Prozeduren beim alltäglichen Tagesverlauf, was einen erheblichen Vorteil gegenüber den im Stand der Technik offenbarten Verfahren und Techniken darstellt. So kann vorteilhafterweise ein Patient oder Anwender beispielsweise während der Arbeit, während einer Freizeitbeschäftigung, beim Schlafen, beim Fernsehen oder bei der Arbeit am PC, auf einem Handy, Smartphone, Tablet-Rechner, das erfindungsgemäße System verwenden. Die Projektionsfläche kann bevorzugt den vollständigen Bildschirm einnehmen, wobei es auch vorteilhaft sein kann, lediglich einen Teil dieser zu verwenden. Vorteilhafterweise befindet sich die Projektionsfläche im Sichtfeld des Anwenders und kann auch im Standby Modus genutzt werden. Vorteilhafterweise kann jeder Display als Projektionsfläche genutzt werden, wodurch das System universell einsetzbar und verwendbar ist. Der Patient kann bevorzugt auch über einen längeren Zeitraum mit dem System verbunden sein, ohne dass dies für ihn körperlich oder psychisch belastend wäre. Vorteilhafterweise können auch alle Arten von Projektionsebenen wie beispielsweise Leinwand, Decke oder Wand kann vorteilhafterweise als Projektionsfläche genutzt werden.

Die Änderung der Signale, insbesondere der optischen Signale erfolgt sinuswellenartig. Es war völlig überraschend, dass sinuswellenartige optische Signale einen Einfluss auf den menschlichen Organismus, insbesondere Organe haben. Die Erfindung betrifft auch die Verwendung sinuswellenartiger optischer Signale zur Beeinflussung elektromagnetischer Schwingungen, biochemischer, hormoneller oder andere Prozesse in einem Körper. Die rhythmische Aktivität eines Organismus zeigt sich vorteilhafterweise auf allen Ebenen. Es ist jedoch bevorzugt, dass die rhythmischen Prozesse mit einer Frequenz oder Intervall von mehreren Sekunden bis zu mehreren Stunden mit der hormonellen/humoralen Regulation und trophischen Prozessen verbunden sind.

Es war völlig überraschend, dass Rhythmen mit einer Frequenz ab 6 bis 30 Sekunden einen Effekt auf Atmungsprozesse und sympatische/parasympatische Aktivitäten oder motorische Aktivitäten zeigen. Weiterhin ist es vorteilhaft, dass Dekasekunden und Minuten Rhythmen beschreiben, die elektrische Aktivität der Hirnrinde zeigen und die Periodik der emotionalen Zustände und Temperaturegulation beeinflussen. Rhythmen mit einer Frequenz von Stunden beeinflussen vorzugsweise den Hypothalamus und die Nierenrinde.

Die Projektionsfläche ist bevorzugt ausgewählt aus der Gruppe umfassend ein Display, ein Monitor, eine Tapete und eine Leinwand. Im Sinne der Erfindung können alle elektronischen Geräte, die eine Projektionsfläche aufweisen, von dem System verwendet werden. Solche elektronischen Geräte umfassen außerdem Handys, MP3-Player, Laptops, Computer, Smartphones, Fernsehgeräte usw. Das System kann hierbei vorteilhafterweise mittels einem Speichermedium auf den entsprechenden elektronischen Geräten wiedergegeben werden. Der Fachmann weiß, dass Speichermedien beispielsweise CDs, Micro-Discs, Flash-Speicher oder sonstige magnetische Speichermedien umfasst.

Der Benutzer kann beispielsweise das System auf seinem Handy oder seinem MP3-Player installieren und es sich während der Fahrt zur Arbeit ansehen oder auf sich optisch wirken lassen. Das System beeinflusst insbesondere das menschliche psychologische und physiologische Verhalten, wobei dieses stimuliert, entspannt und korrigiert wird. Es kann weiterhin für Übungs-, Lern-, Meditations-, und Werbungszwecke verwendet werden. Hierbei wird der bewusste Gedankenablauf verlangsamt, was wiederum zur Vertiefung des Denkens führt und Grundlage für alle Meditationstechniken ist. Versuche haben weiterhin gezeigt, dass hierdurch eine effektive Art zum Lernen und des intellektuellen Trainings bereitgestellt werden kann. Außerdem können mit dem erfindungsgemäßen System die tranceähnlichen Zustände der Psyche erreicht werden. Weiterhin hat sich gezeigt, dass das erfindungsgemäße System großflächig auf Tapeten oder Wänden darstellen lässt und als Verzierung verwendet wird. Es können in Abhängigkeit der Tageszeit- oder Jahreszeit unterschiedliche Bilder als exogene Zeitgeber angezeigt werden.

Es ist bevorzugt, dass sich die Farbe, der Ton, die Farbdichte/Farbsättigung, die Helligkeit und/oder die Intensität der Signale ändern Außerdem kann sich vorteilhafterweise auch der Inhalt eines Bildes ändern. Die Änderung kann beispielsweise eine Bewegung von einem oder mehrerer Punkte oder filmartige Änderungen, wie z. B. ein Sonnenaufgang am Strand umfassen.

Es ist so eine einfache und schnelle Anpassung möglich, wobei die minimale wahrnehmbare Unterscheidungsstufe von jedem Parameter für das Sehvermögen insbesondere 1% der ursprünglichen Größe beträgt.

Die Rhythmen könne vorteilhafterweise in ihrer Frequenz geändert werden. Außerdem kann die Superposition der Rhythmen verändert werden, so dass mehrere Rhythmen überlagert werden. Es ist auch bevorzugt, die bestimmte endogene Rhythmen in bestimmten Tageszeiten anzuwenden, um hierdurch einen stimulierenden und/oder entspannenden Effekt auf einen Betrachter oder Anwender auszuüben. Die Rhythmen können bevorzugt in endogene oder exogene Rhythmen unterteilt werden. Die Phasenänderung der Rhythmen (d. h. ob sich die Akrophase verkürzt oder verlängert), kann ebenfalls als bevorzugte Ausführungsform verwendet werden.

Es ist demgemäß bevorzugt, dass die mehreren optischen Signale mit unterschiedlichen Rhythmen hintereinander gezeigt werden Es kann auch bevorzugt sein, mehrere Rhythmen aufeinander zu legen (Superposition) oder die gegebenen Rhythmen bezüglich innerer Rhythmen des Organismus zu verschieben, um vorteilhafterweise eine Resonanz oder Interferenz zu erreichen. Die optischen Signale können vorteilhafterweise durch alle künstlichen Lichtquellen erzeugt werden. Hierzu zählen unter anderem Lampen, Beleuchtung von elektronischen Geräten, Ambilight bei Fernsehgeräten oder sonstige Lichtquellen. Die optischen Signale sind vorteilhafterweise exogene Zeitgeber. Exogene Zeitgeber bezeichnen im Sinne der Erfindung insbesondere alle im Tagesablauf schwankenden Umweltbedingungen (bspw. Licht, Temperatur, oder Luftfeuchtigkeit). Als exogene Zeitgeber können jedoch auch soziale Zeitgeber, wie z.B. das Verhalten der Artgenossen bezeichnet werden. Die exogenen oder äußeren Zeitgeber werden vorteilhafterweise von den Augen aufgenommen und zur Zirbeldrüse weitergeleitet. Diese produziert tagesrhythmisch das Hormon Melatonin, das den Wach- Schlafrhythmus beeinflusst. Als endogene Zeitgeber wird vorzugsweise die sogenannte innere Uhr bezeichnet, die in jeder Zelle die gesamten Stoffwechselprozesse in Abhängigkeit von der Zeit (Tages-, Schlafrhythmus) beeinflusst. Im Energiestoffwechsel der Zelle können periodische Zyklen beobachtet werden, die vermutlich die Grundlage für die zeitliche Steuerung für Entwicklung und Verhalten von Personen darstellen.

Es bevorzugt, dass die Wahrnehmung der rhythmischen sinuswellenartigen Änderungen bei dauerhafter Anwendung zu einer Phasenverschiebung eines endogenen Vorgangs führt. Mittelwellige Rhythmen, deren Perioden Minuten oder Stunden dauern, können bei Organen, Organsystemen oder Systemen der inneren Sekretion aber auch bei Vorgängen wie Verdauung, Transport von rhythmischer Verteilung der Nährstoffe oder die rhythmische Freisetzung von Hormonen festgestellt werden. Es war völlig überraschend, dass das erfindungsgemäße System, insbesondere die sinuswellenartigen Änderungen, endogene Vorgänge beeinflussen können.

Die minimalen wahrnehmbaren Unterscheidungsstufen von jedem Parameter (insbesondere Farbsättigung, Farbintensität und Helligkeit) für das Sehvermögen betragen einen hundertstel Teil von der ursprünglichen Größe. Die minimalen räumlichen Unterscheidungsmöglichkeiten betragen eine Winkelminute und die minimalen Unterscheidungsmöglichkeiten für die Bewegung sind zwei Winkelsekunden pro Sekunde. Es kann auch bevorzugt sein, dass die Farbabbildung ihre Farbe mit einer Geschwindigkeit von weniger als 5 Nanometer pro Sekunde ändern.

Es hat sich überraschenderweise herausgestellt, dass eine Verwendung des erfindungsgemäßen Systems zur Entspannung, Abbau von Ermüdungserscheinungen und/oder Verbesserung der Konzentration führt.

Im Folgenden soll die Erfindung anhand eines Beispiels erläutert werden, ohne jedoch auf das Beispiel begrenzt zu sein.

### Beispiel 1

Ein Patient schaut sich auf einer Projektionsfläche einen Film an, der die Änderung einer Farbe (z. B. blau) darstellt. Der Farbton variiert innerhalb einer Zeit von 15, 30, 45 oder 60 Sekunden. Die Farbe kann sich beispielsweise von hell nach dunkel verändern. Der Patient fühlte nach der Betrachtung einen Entspannungszustand.

### Beisiel 2

Drei Patienten haben innerhalb von 10 Minuten einen Film angesehen, wobei sich der Farbton von von hell- bis dunkelblau subsensorisch geändert hat (20 Zycklen pro Film). Eine Kontrollgruppe von ebenfalls drei Patienten betrachtete den gleichen Film, bei dem die Änderungen bemerkbar waren. Der Entspannungseffekt der Patienten, die den Film mit den subsensorischen Änderungen betrachteten war deutlich stärker, als bei der Kontrollgruppe.

### Beispiel 3

Ein Patient betrachtet auf einer Projektionsfläche einen Film, der einen Sonnenaufgang am Strand zeigt. Der Film dauert circa 3-4 Minuten. Zu dem Film können akustische Signale abgespielt werden, die dem Betrachter vermitteln, er würde sich an einem Strand befinden. Die Signale können Wassergeräusche, Vögel oder Menschenstimmen umfassen. Der Patient fühlte sich nach der Betrachtung des Films erheblich entspannt.

### Beispiel 4

14 Teilnehmer (männlich/weiblich) im Alter zwischen 25 bis 50 Jahren wurden 5 Minuten dem erfindungsgemäßen System ausgesetzt. Das heißt, die Teilnehmer betrachteten eine Projektionsfläche, auf der unbemerkbare Änderungen stattfanden. Zusätzlich wurden die Teilnehmer einem Kontrollverfahren ausgesetzt, bei dem sie 5 Minuten lang ein Bild mit sichtbaren Änderungen auf einer Projektionsfläche betrachteten. Zwischen den Intervallen beider Verfahren lag mindestens eine Zeitspanne von 4 Stunden. Nach den Verfahren mussten die Teilnehmer den Entspannungseffekt als stark, mäßig oder unbemerkbar einschätzen. Folgendes Ergebnis konnte erzielt werden:

| | Starker Effekt | Mäßiger Effekt | Kein Effekt |
|---|---|---|---|
| Kontrollverfahren | 35% | 50% | 14% |
| System | 57% | 42% | 0% |

Das Ergebnis zeigt, dass 57% der Teilnehmer durch das erfindungsgemäße System einen starken Entspannungseffekt verspürten. Dahingegen beurteilten lediglich 35% der Teilnehmer das Kontrollverfahren als stark entspannend.

## Patentansprüche

1. System zur Beeinflussung eines physiologischen und/oder psychologischen Vorgangs umfassend eine optische Signale wiedergebende Projektionsfläche, **dadurch gekennzeichnet, dass**
die Projektionsfläche so ausgerichtet ist, dass ein Patient die optische Signale auf dieser wahrnehmen kann und wobei die optischen Signale sich rhythmisch und sinuswellenartig ändern, wobei die Änderungen mit einer Frequenz von 6 Sekunden bis 12 Stunden, bevorzugt 6 Sekunden bis 1 Stunde, besonders bevorzugt 10 Sekunden bis 15 Minuten, erfolgen.

2. System nach Anspruch 1, wobei die Projektionsfläche ausgewählt ist aus der Gruppe umfassend ein Display, ein Monitor, eine Tapete und eine Leinwand.

3. System nach Anspruch 1 oder 2, wobei die optischen Signale ausgewählt sind aus der Gruppe umfassend gegenständliche Abbildungen, Farben oder Projektionen unterschiedlicher Lichtintensitäten.

4. System nach mindestens einem der vorhergehenden Ansprüche, wobei mehrere optische Signale mit unterschiedlichen Rhythmen hintereinander gezeigt werden.

5. System nach mindestens einem der vorhergehenden Ansprüche, wobei die optischen Signale exogene Zeitgeber sind.

6. System nach mindestens einem der vorhergehenden Ansprüche, wobei die Wahrnehmung der rhythmischen sinuswellenartigen Änderungen bei dauerhafter Anwendung zu einer Phasenverschiebung eines endogenen Vorgangs führt.

7. System nach mindestens einem der vorhergehenden Ansprüche, wobei die gegenständlichen Abbildungen sich mit einer Geschwindigkeit von weniger als 2, bevorzugt weniger als 1 Winkelsekunde pro Sekunde ändern und/oder die Farbabbildungen ihre Farbe mit einer Geschwindigkeit von weniger als 5 nm pro Sekunde ändern.

8. Verwendung des Systems nach mindestens einem der vorhergehenden Ansprüche, zur subsensorischen Stimulation eines Patienten.

9. Verwendung des Systems nach mindestens einem der vorhergehenden Ansprüche, zur Entspannung, Abbau von Ermüdungserscheinungen und/oder Verbesserung der Konzentration.
